(19) **Europäisches Patentamt**
**European Patent Office**
**Office européen des brevets**

(11) **EP 1 485 506 B1**

(12) **EUROPEAN PATENT SPECIFICATION**

(45) Date of publication and mention
of the grant of the patent:
**08.07.2009 Bulletin 2009/28**

(21) Application number: **03743886.8**

(22) Date of filing: **11.03.2003**

(51) Int Cl.:
*C12Q 1/68* (2006.01)    *C07H 21/00* (2006.01)

(86) International application number:
**PCT/EP2003/002879**

(87) International publication number:
**WO 2003/076657 (18.09.2003 Gazette 2003/38)**

(54) **METHOD FOR IDENTIFYING ANIMALS FOR MILK PRODUCTION QUALITIES BY ANALYZING THE POLYMORPHISM OF THE PIT-1 AND KAPPA-CASEIN GENES**

VERFAHREN ZUR IDENTIFIZIERUNG DER MILCHPRODUKTIONSQUALITÄTEN IN TIEREN DURCH ANALYSE DER PIT-1 UND KAPPA-KASEIN GENPOLYMORPHISMUS

PROCEDE D'IDENTIFICATION DES QUALITES DE LA PRODUCTION LAITIERE DES ANIMAUX PAR ANALYSE DU POLYMORPHISME DES GENES PIT-1 ET DE KAPPA CASEINE

(84) Designated Contracting States:
**AT BE BG CH CY CZ DE DK EE ES FI FR GB GR HU IE IT LI LU MC NL PT RO SE SI SK TR**
Designated Extension States:
**LT LV**

(30) Priority: **11.03.2002 FR 0203026**

(43) Date of publication of application:
**15.12.2004 Bulletin 2004/51**

(73) Proprietors:
• **Arysta LifeScience Europe**
**91140 Villebon sur Yvette (FR)**
• **Patrimoine de la Faculté Universitaire des Sciences Agronomiques de Gembloux**
**5030 Gembloux (BE)**

(72) Inventors:
• **RENAVILLE, Robert**
**B-5030 Gembloux (BE)**
• **GENGLER, Nicolas**
**B-5190 Jemeppe sur Sambre (BE)**

(74) Representative: **Desaix, Anne et al**
**Ernest Gutmann - Yves Plasseraud S.A.S.**
**3, rue Auber**
**75009 Paris (FR)**

(56) References cited:
**EP-A- 0 821 070**    **US-A- 5 614 364**

• **RENAVILLE R ET AL: "Pit-1 gene polymorphism, milk yield, and conformation traits for Italian Holstein-Friesian bulls" JOURNAL OF DAIRY SCIENCE, vol. 80, no. 12, December 1997 (1997-12), pages 3431-3438, XP002261251 ISSN: 0022-0302**
• **VELMALA R J ET AL: "A search for quantitative trait loci for milk production traits on chromosome 6 in Finnish Ayrshire cattle" ANIMAL GENETICS, vol. 30, no. 2, April 1999 (1999-04), pages 136-143, XP002261252 ISSN: 0268-9146**
• **PARMENTIER I ET AL: "Candidate gene markers associated with somatotropic axis and milk selection" DOMESTIC ANIMAL ENDOCRINOLOGY, vol. 17, no. 2-3, October 1999 (1999-10), pages 139-148, XP001154779 ISSN: 0739-7240**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to the fields of milk production and breeding. M ore particularly, this invention relates to methods a nd kits for determining the milk production potential in an animal from an analysis of the polymorphism of its Pit-1 and κ-casein genes.

Currently, selecting a particular trait in an animal is slow and costly. Said selection is based in part on observation of that animal by dint of different types of measurement such as its weight, size, colour etc., and partly on a study of its genealogy.

Said selection can be made over several generations by crossing animals with the same desired trait and hoping that that trait will be dominant in the next generation.

That type of selection has a number of disadvantages due to the length of time required and also because the judgment of an expert breeder in the field is required.

The breeder's judgment is based on observations and hypotheses and thus includes a degree of subjectivity linked with experience. Further, a trait selected in that manner in a given generation will not necessarily be transmitted to the next generation.

To overcome the disadvantages mentioned above, novel methods are being developed that are based on scientific advances made in the genetics field.

A particular promising approach consists of studying genes with large individual effects and their possible association with certain desired traits in the animals. Such genes can then be used as molecular markers to select the traits in question in an animal. That method necessitates identifying genes with large individual effects or genetic markers associated with the desired traits. The success of that approach hence depends on demonstrating a correlation between the phenotypic trait sought and a certain polymorphism of the gene in question.

In the milk production field, a number of genes have been identified as having an influence on milk production, both in terms of the quantity produced (for example genes of the somatotropic axis) and in terms of milk quality (for example genes associated with protein variations).

A number of genes associated with the lactodynamographic properties of milk have been identified, in particular the kappa-casein gene (see the review article by Formaggioni et al., 1999). In particular, the influence of polymorphism in the κ-casein gene on Holstein cow milk production traits has been widely demonstrated in the literature (Van Eenennaam and Medrano, 1991; Bovenhuis et al., 1992; Mao et al., 1992; Ron et al., 1994). Allele B of said gene is associated with a milk lactodynamographic quality that is favourable for cheese production, which means that more cheese can be produced, with a firmer curd and a shorter curd formation time (Martinet and Houdebine, 1993).

The influence of different genes of the somatotropic axis on milk production has also been described. As an example, supplying growth hormone is known to stimulate milk production (see the article by Chilliard et al., 2001). A further somatotropic gene, the Pit-1 gene, has also been identified as having an influence on the quantity of milk produced. In PCT patent application WO-A-98/03677 filed on 22nd July 1996, the inventors demonstrated that allele A of the Pit-1 gene was associated with advantageous milk production traits while allele B was associated with better meat production. Those two alleles differ by substitution of an adenine (allele A) by a guanine (allele B) at position 1178.

The aim of the invention is to provide a method for identifying a mammal having a genotype indicative of advantageous milk production traits. This method should enable the identification of high potential for milk production and protein production in milk in a mammal, and perform better than known prior art methods. In the remainder of the text, the different alleles of the κ-casein gene will be designated in the same manner as in the scientific literature of the prior art, as described in Table 4 of the article by Formaggioni et al., see above. Examples of methods for demonstrating the presence of alleles A and B of the κ-casein gene are described in Examples 1 and 2 below by Restriction Fragment Length Polymorphism (RFLP) and by allele-specific detection, respectively.

Alleles A and B of the Pit-1 gene are those described in PCT patent application WO-A-98/03677. The inventors have also demonstrated a further polymorphism in the Pit-1 gene in the 2 exon (Example 5). The corresponding alleles C and T differ by substitution of an adenine (allele C) by a guanine (allele T) in the codon for Serine 65. That mutation, while silent, can act as a molecular marker associated with milk production traits. The inventors have observed that in 97.8% of bulls tested (in a sample of more than 500 Holstein bulls), the AA genotype for exon 6 is associated with the TT genotype for exon 2, while the BB genotype for exon 6 and CC genotype for exon 2 are correlated. The association between the genotype observed for said mutation and the milk production performance is of the same order as for exon 6.

The Pit-1 gene and the κ-casein gene do not belong to the same metabolic chain. Thus, a priori, they do not have any reason to interact.

Nevertheless, the inventors have developed a method for identifying a mammal that has a genotype indicative of advantageous milk production traits, in which both the Pit-1 gene and the kappa-casein gene are used as genetic markers. They have shown that this method is more effective than known prior art methods based on the use of the Pit-1 marker alone or on the kappa-casein marker alone.

In a first aspect, the invention concerns a method for identifying a mammal having a genotype that is indicative of advantageous milk production traits, comprising the following steps:

a) obtaining a biological sample (or tissue) comprising the DNA of said mammal;
b) analyzing the polymorphism of the Pit-1 and κ-casein genes of said mammal, in which the simultaneous presence of allele A and/or T of the Pit-1 gene and allele B of the κ-casein gene is indicative of high potential for milk production and protein production in said mammal.

In a preferred implementation of this method, the mammal is a bovine.

Using a detailed statistical method described in Example 6 below, the inventors have demonstrated that animals with $AA_{Pit-1}$ and $BB_{\kappa-casein}$ genotypes have far superior milk production performances than those with bovine $BB_{Pit-1}AA_{\kappa-casein}$ genotypes. As summarized in particular in Table 3, cows tested as $AA_{Pit-1}$ and $BB_{\kappa-casein}$ produce about 237 kg more milk on average, on the basis of 305 days of lactation, than those tested $BB_{Pit-1}$ and $AA_{\kappa-casein}$. This effect is greater than the sum of the effects observed for the two genes individually (46.3 kg for Pit-1 and 72.2 for κ-casein). Similarly, a very large effect of an association of the two favourable alleles is observed on protein production, which effect is greater than the sum of the individual effects of the two genes.

The results shown in Example 7, obtained on a smaller sample of animals, show that an analysis of Pit-1 gene polymorphism can also be carried out at exon 2, in which case it is the simultaneous presence of allele T of the Pit-1 gene and allele B of the κ-casein gene that is indicative of high milk production and protein production potential in the test animal.

Results published by Chilliard et al. (2001) show that administration of a large quantity of growth hormone does not significantly modify the amount of proteins produced in the milk. Since Pit-1 and growth hormone belong to the same metabolic axis, Chilliard's results suggest that Pit-1 does not exert a direct effect on the amount of proteins produced by the mammary gland. The total quantity of proteins, expressed in kg for complete lactation, can be modified, however, following a modification in the volume of the milk produced.

In the methods of the invention, the presence of allele B of κ-casein is also indicative of a lactodynamographic milk quality that is favourable to cheese production.

The first step in the methods of the invention, i.e., obtaining a biological sample comprising the DNA of the animal to be tested, can be carried out using any technique that is known to the skilled person, for example by means of a biopsy, or removing a blood sample or any other biological sample. Preferably, this step is carried out using cells deriving from hair follicles obtained by plucking a few hairs from the animal.

Of course, the above steps 1 and 2 can be carried out by 2 different persons. For example, the sample can be obtained by the breeder and then sent to a laboratory that will carry out the analysis. Therefore, the present invention also concerns a method for identifying a mammal having a genotype that is indicative of advantageous milk production traits, comprising analyzing in a biological sample from said bovine the polymorphism of the Pit-1 and κ-casein genes of said mammal, in which the simultaneous presence of allele A and/or T of the Pit-1 gene and allele B of the κ-casein gene is indicative of high potential for milk production and protein production in said mammal.

In the methods of the invention, the polymorphism in the κ-casein gene can be analyzed by restriction fragment length polymorphism (RFLP), by amplifying a fragment comprising nucleotide 5345 of the sequence described by Alexander et al., 1988 (which corresponds to A in allele A and C in allele B) of the κ-casein gene and by digesting the product of this amplification with the restriction enzyme Hinfl. As described in Example 1 below, such a fragment can be amplified using the following primers:

5'-ATCATTTATGGCCATTCCACCAAAG-3' (SEQ ID No: 1) and
5'-GCCCATTTCGCCTTCTCTGTAACAGA-3' (SEQ ID No: 2).

Alternatively and preferably, the analysis is implemented for the κ-casein gene by allele-specific amplification and/or detection. Example 2 illustrates the allele-specific detection technique carried out with the following primers and probes:

Kappa F primer: 5'-CCGAAGCAGTAGAGAGCACTGTAG-3' (SEQ ID No: 3);
Kappa R primer: 5'-TCTCAGGTGGGCTCTCAATAACTT-3' (SEQ ID No: 4);
Kappa Vic probe: 5'-TACTCTAGAAGATTCTC-3' (SEQ ID No: 5);
Kappa Fam probe: 5'-TACTCTAGAAGCTTCTC-3' (SEQ ID No: 6).

Similarly, in the methods of the invention, the analysis can be carried out for the Pit-1 gene by restriction fragment length polymorphism analysis (RFLP), by amplifying a fragment comprising nucleotide 1178 of the Pit-1 gene and digesting the product of said amplification with the restriction enzyme Hinfl, as shown in Example 3 below. To this end, the following primers can be used:

5'-AAACCATCATCTCCCTTCTT-3' (SEQ ID No: 7);
5'-AATGTACAATGTGCCTTCTGAG-3' (SEQ ID No: 8).

In the methods of the invention, the analysis can also be carried out, for the Pit-1 gene, by allele-specific amplification and/or detection. Examples 4 and 5 below illustrate allele-specific amplification of Pit-1 gene fragments, respectively in exons 6 and 2, with the following primers:

- for allele-specific amplification at exon 6:

    5'-CAGAGAGAAAAACGGGTGAAGACAAGCATG-3' (SEQ ID No: 9);
    specific for allele B;
    5'-CAGAGAGAAAAACGGGTGAAGACAAGCATA-3' (SEQ ID No: 10);
    specific for allele A; and
    5'-AGATAGAGGGAAAGATATAGTGAAAGGGACAG-3' (SEQ ID No: 11);
    as the reverse primer;

- for allele-specific amplification at exon 2:

    5'-C TGC CAT CAC GCC ATA GTT C-3' (SEQ ID No: 12.),
    specific for allele C;
    5'- C TGC CAT CAC GCC ATA GTT T-3' (SEQ ID No: 13),
    specific for allele T; and
    5'-CA ACA GGA CTT CAT TAT TCT GTT CCT CAT TAT TCT GTT CCT T -3' (SEQ ID No: 14) as the reverse primer.

Polymorphism in the P it-1 gene can also be determined by allele-specific detection of an amplified fragment of said gene, for example at exon 6, using the following probes and primers:

    PIT-1 F primer: 5'-CATTCGAGATGCTCCTTAGAAATAGTAA-3' (SEQ ID No: 15);
    PIT-1R primer: 5'-GTTTTGTAACCGAAGGCAGAGAGA-3' (SEQ ID No: 16);
    PIT-1MGB FAM probe: 5'-AACTCTGATTTAGGCTTG-3' (for allele A) (SEQ ID No: 17); and
    PIT-1MGB VIC probe: 5'-AACTCTGATTCAGGCTT-3' (for allele B) (SEQ ID No: 18).

Clearly, the experimental conditions described in Examples 1 to 5 are provided solely by way of indication, and can be modified by the skilled person. This is particularly the case as regards the reagents and the temperature conditions used to carry out the amplification reactions, but also for the primer sequences. Knowing the position of the polymorphism that is to be detected, it is easy to determine other primers or probes that can be used to amplify the fragment concerned and identify the allele in question. Said primer determination can be made by reading the sequence surrounding the polymorphism site, if necessary using software of the GeneScan® type (Applied Biosystems).

Further, other techniques can be used to determine polymorphism in the genes under consideration, such as techniques known as SSCP (single stranded conformation polymorphism), DGGE (denaturing gradient gel electrophoresis) and CFLP (cleavage fragment length polymorphism), which have been described, for example, by Sambrook et al. (Molecular Cloning - A Laboratory Manual, Third Edition - Cold Spring Harbor Laboratory Press).

The invention also concerns a kit for identifying a genotype indicative of advantageous milk production traits in cattle, comprising oligonucleotides for amplifying a fragment comprising nucleotide 1178 of the Pit-1 gene, oligonucleotides for amplifying a fragment comprising nucleotide 5345 of the κ-casein gene, and the restriction enzyme Hinfl.

As an example, such a kit can contain the following primers to amplify a fragment comprising nucleotide 1178 of the Pit-1 gene:

    5'-AAACCATCATCTCCCTTCTT-3' (SEQ ID No: 7); and
    5'-AATGTACAATGTGCCTTCTGAG-3' (SEQ ID No: 8);

and the following primers to amplify a fragment comprising nucleotide 5345 of the κ-casein gene:

    5'-ATCATTTATGGCCATTCCACCAAAG-3' (SEQ ID No: 1) and
    5'-GCCCATTTCGCCTTCTCTGTAACAGA-3' (SEQ ID No: 2).

In a further aspect, the present invention concerns a kit for identifying a genotype indicative of advantageous milk production traits in cattle, comprising oligonucleotides for carrying out allele-specific amplification and/or detection of a fragment of the Pit-1 gene, and oligonucleotides for carrying out allele-specific amplification and/or detection of a fragment of the κ-casein gene.

In a preferred implementation of such a kit, the following primers constitute the oligonucleotides for allele-specific amplification of a fragment of the Pit-1 gene:

5'-CAGAGAGAAAAACGGGTGAAGACAAGCATG-3' (SEQ ID No: 9); specific for allele B;
5'-CAGAGAGAAAAACGGGTGAAGACAAGCATA-3' (SEQ ID No: 10); and specific for allele A; and
5'-AGATAGAGGGAAAGATATAGTGAAAGGGACAG-3' (SEQ ID No: 11); as the reverse primer.

In an alternative implementation of such a kit, the following primers constitute the oligonucleotides for allele-specific amplification of a fragment of the Pit-1 gene targeting exon 2:

5'-C TGC CAT CAC GCC ATA GTT C-3' (SEQ ID No: 12),
specific for allele C;
5'- C TGC CAT CAC GCC ATA GTT T-3' (SEQ ID No: 13),
specific for allele T; and
5'-CA ACA GGA CTT CAT TAT TCT GTT CCT CAT TAT TCT GTT CCT T -3' (SEQ ID No: 14) as the reverse primer.

In a further implementation of a kit of the invention, the following primers and probes constitute the oligonucleotides for allele-specific detection of a fragment of a gene for κ-casein:

Kappa F primer: CCGAAGCAGTAGAGAGCACTGTAG (SEQ ID No: 3);
Kappa R primer: TCTCAGGTGGGCTCTCAATAACTT (SEQ ID No: 4);
Kappa Vic probe: TACTCTAGAAGATTCTC (SEQ ID No: 5);
Kappa Fam probe: TACTCTAGAAGCTTCTC (SEQ ID No: 6);

and the following primers and probes constitute the oligonucleotides for allele-specific detection of a fragment of the Pit-1 gene:

PIT-1 F primer: CATTCGAGATGCTCCTTAGAAATAGTAA (SEQ ID No: 15);
PIT-1R primer: GTTTTGTAACCGAAGGCAGAGAGA (SEQ ID No: 16);
PIT-1MGB FAM probe: AACTCTGATTTAGGCTTG (for allele A) (SEQ ID No: 17); and
PIT-1MGB VIC probe: AACTCTGATTCAGGCTT (for allele B) (SEQ ID No: 18).

Clearly, the skilled person can use other nucleotide sequences, whether for amplifying fragments of the κ-casein and Pit-1 gene, or for their detection. Similarly, the skilled person will be able to modify the labeling of the probes used.

The application also concerns a genetic marker for determining the milk or meat capacity cattle, **characterized in that** an adenine in position 195 of the Pit-1 gene is characteristic of good milk production and a guanine in position 195 of the Pit-1 gene is characteristic of good meat production.

The following examples and figures provide non-limiting illustrations and details of certain aspects of the present invention.

## LEGENDS TO THE FIGURES

Figure 1 shows the results of allele-specific amplification of exon 6 of the Pit-1 gene, from genomic DNA of an AA homozygous animal (sample 1), a BB homozygous animal (sample 2) and an AB heterozygous animal (sample 3).
Figure 2 shows the primers used to carry out allele-specific amplification applied to exon 2 of the Pit-1 gene. The gene sequence is shown in italics.
Figure 3 shows the result of allele-specific amplification of exon 2 of the Pit-1 gene from genomic DNA of a CC homozygous animal (sample 1), a TT homozygous animal (sample 2) and a CT heterozygous animal (sample 3).

## EXAMPLE 1: Determination of A and B alleles for kappa-casein by RFLP analysis

The reaction mixture was composed of $H_2O$, 10x buffer, 2 mM of $MgCl_2$, 20 pmol of primers, 0.2 mM of dNTPs,

100 ng/25μl of DNA and 0.6U/25μl of polymerase [Goldstar DNA Polymerase, EUROGENTEC]. The PCR reaction cycle was constituted by a first 3 minutes denaturing phase at 96°C followed by 40 cycles of 1 minute at 94°C, 1 minute at 66°C and 1 minute at 72°C. The terminal extension phase was carried out at 72°C for 10 minutes.

The PCR product was then incubated with 20U of the Hinfl enzyme for 2 hours at 37°C. Following incubation, 2 μl of STOP solution (50% glycerol, 20 mM of EDTA, 0.25% of bromophenol blue) was added and the mixture was separated by 2% agarose gel electrophoresis.

> Primers: 5'-ATCATTTATGGCCATTCCACCAAAG-3'
> 5'-GCCCATTTCGCCTTCTCTGTAACAGA-3'.

The allele arbitrarily designated "A" in the scientific literature was cut by the Hinfl enzyme and the "B" allele was not digested by the Hinfl enzyme.

## EXAMPLE 2: Determination of A and B alleles for kappa-casein by allele-specific detection

The reaction mixture was composed of Taq Man Universal PCR master mix (Applied Biosystems Inc) in the presence of primers and probes:

| | |
|---|---|
| Kappa F primer | 2.5 μl |
| Kappa R primer | 900 nM |
| Fam Kappa probe | 200 nM |
| Vic Kappa probe | 200 nM |

in a volume made up to 5 μl with water.

Amplification was measured in real time using an Applied Biosystems model 7900 HT apparatus.

The PCR reaction cycle was constituted by a first phase of one cycle at 50°C for 2 minutes followed by 1 cycle of 10 min at 95°C followed by 40 cycles of 15 seconds at 95°C and 1 minute at 60°C.

The results were read automatically by the apparatus.

**Primers used:**

F primer: CCGAAGCAGTAGAGAGCACTGTAG (SEQ ID No: 3); and
R primer: TCTCAGGTGGGCTCTCAATAACTT (SEQ ID No: 4);

**Probes:**

Vic probe: TACTCTAGAAGATTCTC (SEQ ID No: 5); and
Fam probe: TACTCTAGAAGCTTCTC (SEQ ID No: 6).

The allele arbitrarily named A in the literature was that with nucleotide A in position 5345 and allele B was that with nucleotide C in position 5345.

## EXAMPLE 3: Determination of A and B alleles for Pit-1 gene by RFLP analysis

Polymorphism of the Pit-1 gene at nucleotide 1178 was analyzed using the following primers:

5'-AAACCATCATCTCCCTTCTT-3' (SEQ ID No: 7); and
5'-AATGTACAATGTGCCTTCTGAG-3' (SEQ ID No: 8)

PCR amplification was carried out under conditions substantially identical to those described in Example 1.

The product of that amplification was a fragment with 451 base pairs. Incubation of that fragment with the restriction enzyme Hinf1 enabled two alleles to be distinguished: allele A was not cut by Hinf1 and allele B comprises a Hinf1 restriction site, which generates two fragments with 244 and 207 base pairs.

## EXAMPLE 4: Determination of A and B alleles of exon 6 of the Pit-1 gene by allele-specific amplification

This method is based on the use, for PCR, of two primers for which the nucleotide located at the terminal 3' position are different. This difference is responsible for the specificity of the primers as regards one of the two alleles of the gene. This method thus uses specific primers for each allele, and does not require the use of a restriction enzyme.

Amplification reactions were carried out in a solution of $H_2O$, 10x buffer, 3 mM $MgCl_2$, 20 p mole/50 $\mu$l of specific primer (A or B), 400 $\mu$m dNTP, 100 ng/50 $\mu$l of DNA, and 1.2 U/50 $\mu$l of polymerase [Goldstar DNA polymerase, EUROGENTEC]. The PCR reaction comprised a first denaturing step carried out at 96°C for 3 minutes, followed by about 35 cycles of 1 minute at 95°C, 1 minute at 65.2°C and 1 minute at 72°C. The final step was carried out at 72°C for 10 minutes.

The primers used in this method were as follows:

5'-CAGAGAGAAAAACGGGTGAAGACAAGCATG-3' (SEQ ID No: 9); specific for allele B;
5'-CAGAGAGAAAAACGGGTGAAGACAAGCATA-3' (SEQ ID No: 10); and specific for allele A; and
5'-AGATAGAGGGAAAGATATAGTGAAAGGGACAG-3' (SEQ ID No: 11); as the reverse primer.

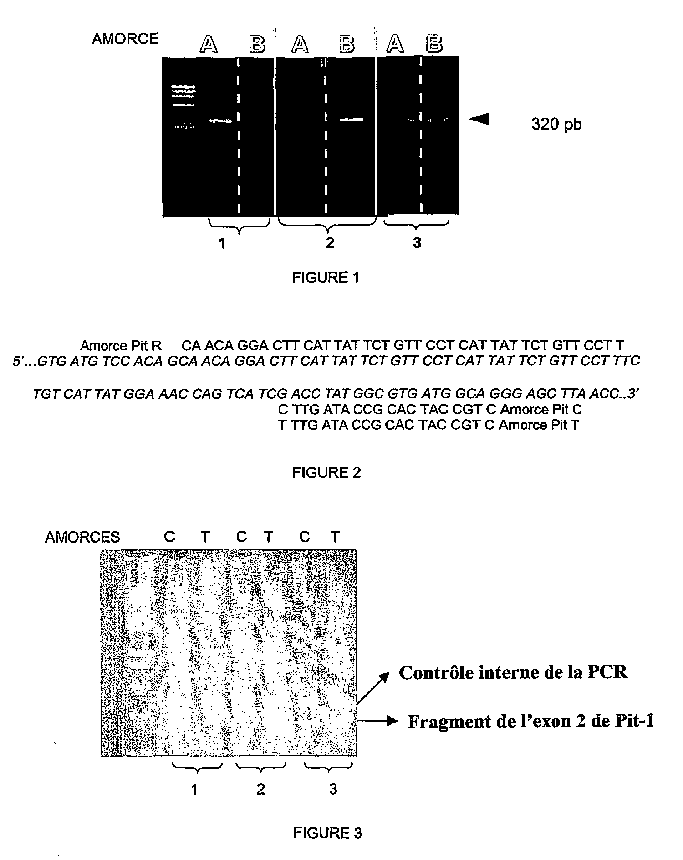

After the amplification reaction, the products obtained were revealed on agarose gel, as shown in Figure 1. In this experiment, each sample was brought into the presence of either primer A or primer B. The PCR amplified 320 bp fragment was either present alone in tube A (the animal was thus of genotype AA) or only present in tube B (the animal was thus a BB homozygote) or it was present in both tubes (the animal was AB heterozygous).

**EXAMPLE 5: Determination of C and T alleles for exon 2 of the Pit-1 gene by allele-specific amplification**

A second polymorphous site was determined using single strand conformational polymorphism (SSCP) at the Pit-1 gene. Sequencing of the polymorphous part of the gene allowed the responsible mutation to be identified. It was an A→G transition at codon 65 of the serine in the protein transactivation region. The amino acid was not modified by this mutation. The two alleles which resulted from this mutation caused the existence of three polymorphous profiles in SSCP.

A study of this point mutation on the sample of Holstein cattle necessitated the development of a rapid analytical method that was easy to carry out, which is not the case with the SSCP method.

An allele-specific PCR reaction was thus developed to study this mutation. One of the primers, Pit C, was specific for allele C. The other, Pit T, was specific to allele T. The Pit R allele was used as the reverse primer. To increase the specificity of the reaction, a supplemental mutation was introduced at the third base upstream of the 3'-OH end of the two specific primers (Figure 2).

Pit-C: 5'-C TGC CAT CAC GCC ATA GTT C-3' (SEQ ID No: 12);
Pit-T: 5'- C TGC CAT GAG GCC ATA GTT T-3' (SEQ ID No: 13); and
Pit-R: 5'-CA ACA GGA CTT CAT TAT TCT GTT CCT CAT TAT TCT GTT CCT T -3' (SEQ ID No: 14).

**EXAMPLE 6: Combination of the effect of the Pit-1 gene (exon 6) with that of the κ-casein gene.**

The genotype for the gene for κ-casein was determined for a sample of 1100 Holstein bulls at the point mutation at nucleotide 5345 by Alexander et al., 1988, who differentiated variant B from variant A. The frequency of genotypes AA, AB and BB were as follows: 75%, 23% and 2% respectively, which corresponds to 86.5% of allele A and 13.5% of allele B. They follow the Hardy-Weinberg law. These frequencies are similar to those obtained by Van Eenennaam and Medrano (1991) from 1152 Holstein cows (82% A and 18% B) and by Ron et al., (1994) from 119 Holstein bulls (78.6% AA, 20.5% AB and 0.9% BB).

The table below (Table 1) shows the results of a statistical study of the association between the polymorphism of the κ-casein gene and milk production traits obtained in the same study, compared with those obtained by Van Eenennaam et al., (1991) and Ron et al., (1994).

**TABLE 1: Comparative table summarizing results obtained on sample of 1100 Holstein Semex Alliance bulls (Canada and by Van Eenennaam et al. (1991), Ron et al. (1994). α: effect on production traits of substitution of allele B by allele A in the κ-casein gene.**

|  | Van Eenennaam et al., (1991) | Ron et al., (1994) | 1100 Holstein bulls |
|---|---|---|---|
| Production characters | α | α | α |
| Milk, kg | -146.0 | -139.3 | -72.2 |

(continued)

| | Van Eenennaam et al., (1991) | Ron et al., (1994) | 1100 Holstein bulls |
|---|---|---|---|
| Fat, kg | -2.0 | -0.28 | -4.2 |
| Proteins, kg | -7.0 | -2.21 | -2.5 |
| Fat, % | -0.044 | -0.044 | -0.02 |
| Proteins, % | -0.022 | -0.022 | -0.001 |

In the three studies, allele B had a positive effect on the milk yields, fat and proteins produced without modifying the percentages of fat and proteins. A comparison of the amplitude of the effects shows that overall, these can be considered to be similar. The observed differences can be due to the sample used, the statistical model used and/or the sample.

SAMPLE

The effects of allele substitution in the different genes studied were calculated from a total of 2397037 lactations of 1094443 daughters of 1100 Canadian Holstein bulls. The official lactation data originate from the "Canadian Dairy Network". Table 2 shows the mean values, standard deviations, maxima and minima calculated for the quantities of milk, fat and proteins produced on the basis of 305 days lactation. The relatively high standard deviations and the relatively broad minimum-maximum range show the diversity of this sample in terms of milk production performance. These values are characteristic of a non-selected sample comprising high performance cattle and others that are less so.

**TABLE 2: Mean values, standard deviations, minima and maxima calculated for different production parameters from descendants from 110 Holstein (Semex) bulls calculated on the basis of 305 lactation days.**

| Production character (kg) | Mean | Standard deviation | Minimum | Maximum |
|---|---|---|---|---|
| Milk | 8110.9 | 1778.1 | 1049.0 | 22135.0 |
| Fat | 300.7 | 68.3 | 37.0 | 971.0 |
| Proteins | 261.1 | 55.6 | 32.0 | 705.0 |

GENETIC MODEL

The allele probabilities were introduced into a simplified version of a Canadian animal model:

$$y = Xh + Tt + Qr + Zp + Z^*a + e$$

In which :

y = vector for 305 days lactation production (milk, fat or proteins);
h = vector for fixed herd-year-season (October to February and March to September) - parity (first or higher) effects;
t = vector for fixed lactation (1 to 6 or more) - age classes - month of lactation effects;
r = vector for 2 regression coefficients on transformed allele probabilities;
p = vector for random permanent environmental effects;
a = vector for random polygenic effects;
e = vector for random residual effects;
X, T, Z and Z* = incidence matrices linking h, t, p and a to y; and
Q = 2-column regression variable matrix, 1 column for each allele probability (Pit-1, K-casein).

The components for the variance used are those used in Canada. They were a heritability of 0.33 and a repeatability of 0.54. The equations were solved using a "preconditioned conjugate gradient" as convergence was very slow. More than 300 iterations were typically necessary.

The difference between the two coefficients was proportional to 2 x the allele substitution effect. This was obtained by a calculation using an arcsine transformation of the regression variables. The results for the fat and protein percentages were obtained indirectly using means of population:

$$\Delta C\% = \frac{100 \times \Delta C - \Delta L \times \overline{C}\%}{\overline{L} + \Delta L}$$

in which:

$\overline{L}$ = mean of milk in the population;
$\Delta L$ = effect on milk;
$\overline{C}\%$= m ean of milk component percentage (fat or protein);
$\Delta C$ = effect on component (quantity);
$\Delta C\%$ = effect on component (percent).

## Results

Table 3 shows the estimated additive effects on milk production traits for 110 Canadian Holstein bulls.

**TABLE 3: Estimated additive effects on milk production traits for 1100 Canadian Holstein bulls. $\alpha^1$ : effect on production traits of substitution of allele B by allele A of the Pit-1 gene. $\alpha^2$: effect on production traits of substitution of allele B by allele A of the $\kappa$-casein gene. $\alpha^3$: effect on production traits of substitution of allele B by allele A of the Pit-1 gene and of substitution of allele A by allele B of the $\kappa$-casein gene.**

|  | Pit-1 (exon 6) | $\kappa$-casein | $AA_{Pit-1}BB_{\kappa-casein}$ - $BB_{Pit-1}AA_{\kappa-casein}$ |
|---|---|---|---|
| Production characters | $\alpha^1$ | $\alpha^2$ | $\alpha^3$ |
| Milk, kg | 46.3 | -72.2 | 237 |
| Fat, kg | 1.5 | -4.2 | 11.4 |
| Proteins, kg | 1.9 | -2.5 | 8.8 |
| Fat, % | -0.0002 | -0.02 | - |
| Proteins, % | 0.004 | -0.001 | - |

A selection of the best alleles of the Pit-1 (allele A) and $\kappa$-casein (allele B) genes could thus lead to a substantial increase in milk quantities, fat and milk proteins, without modifying the percentages of proteins and fat in milk.

### EXAMPLE 7: Combination of the effect of Pit-1 (exon 2) with that of $\kappa$-casein.

The animal model and the statistical model were identical to those described in the preceding example. Table 4 summarizes the observed results:

**TABLE 4: Estimated additive effects on milk production traits for 1100 Canadian Holstein bulls. $\alpha^1$ : effect on production traits of substitution of allele C by allele T of the Pit-1 gene. $\alpha^2$: effect on production traits of substitution of allele B by allele A of the $\kappa$-casein gene. $\alpha^3$: effect on production traits of substitution of allele C by allele T of the Pit-1 gene and of substitution of allele A by allele B of the $\kappa$-casein gene.**

|  | Pit-1 (exon 2) | K-casein | $TT_{Pit-1}BS_{\kappa-casein}$ - versus $CC_{Pit-1}AA_{\kappa-casein}$ |
|---|---|---|---|
| Production characters | $\alpha^1$ | $\alpha^2$ | $\alpha^3$ |
| Milk, kg | 17.11 | -21.41 | 77.04 |
| Fat, kg | 1.57 | -1.21 | 5.56 |

(continued)

| | Pit-1 (exon 2) | K-casein | TT$_{Pit-1}$BS$_{\kappa\text{-casein}}$ - versus CC$_{Pit-1}$AA$_{\kappa\text{-casein}}$ |
|---|---|---|---|
| Proteins, kg | 1.18 | -0.14 | 2.64 |
| Fat, % | 0.015 | -0.01 | - |
| Proteins, % | 0.011 | -0.001 | - |

It can be seen that using allele T from the Pit-1 gene and allele B from the kappa-casein gene simultaneously in accordance with the invention results in a substantial increase in milk, fat and milk protein quantities without modifying the percentage of proteins and fat in the milk.

**REFERENCES**

(1) Alexander et al. (1988) Eur. J. Biochem., 178 (2), 395-401. Accession number: X14908, X14326

(2) Bovenhuis S., Van Arendonk J., Korver S. (1992). Association between milk protein polymorph isms and milk traits. J. Dairy Sci., 75 : 2549-2559.

(3) Chilliard et al, Biotechnology in Animal Husbandry, Renaville R. & Burny A. (eds), Kluwer academic publishing, pages 65-97 May 2001.

(4) Formaggioni et al. (1999) Milk protein polymorphism: detection and diffusion of the genetic variants in bos genus. Annali della Facolta di Medicina Veterinaria Vol. XIX

(5) Mao I., Buttanozzi L.G., Aleandri R. (1992). Effects of polymorphic milk protein genes on milk yield and composition traits in Holstein cattle. Acta Agric. Scand., Sect. A, Animal Sci., 42: 1-7.

(6) Martinet et Houdebine (1993) Biologie de la lactation Editions Inserm, Inra Editions 587 pages.

(7) Ron M., Yoffe O., Ezra E., Medrano J.F., Weller (1994). Determination of effects of milk protein genotype on production traits of Israeli Holsteins. J. Dairy Sci., 77:1106-1113.

(8) Sambrook et al. (Molecular Cloning - A laboratory manual. Third Edition - Cold Spring Harbor Laboratory Press.

(9) Van Eenennaam A., Medrano J.F. (1991). Milk protein polymorphisms in California dairy cattle. J. Dairy Sci., 74: 1730- 1742.

(10) EP 96 401 634.9, filed on 22nd July 1996.

**Claims**

1. A method for identifying a bovine having a genotype that is indicative of advantageous milk production traits, comprising analyzing in a biological sample from said bovine the polymorphism of the Pit-1 and κ-casein genes of said bovine, in which the simultaneous presence of allele A and/or T of the Pit-1 gene and allele B of the κ-casein gene is indicative of high potential for milk production and protein production in said bovine,
said allele A of the Pit-1 gene being **characterized by** the presence of adenine at position 1178 of the Pit-1 gene,
said allele T of the Pit-1 gene being **characterized by** the presence of guanine at position 195 of the Pit-1 gene,
said allele B of the κ-casein gene being **characterized by** the presence of cytosine at position 5345 of the κ-casein gene.

2. An identification method according to claim 1, in which the presence of allele B of the κ-casein is also indicative of a lactodynamographic, milk quality that is favourable to cheese production.

3. An identification method according to claim 1 or 2, in which said biological sample comprises hair follicle cell DNA of said bovine.

4. An identification method according to any one of claims 1 to 3, in which said polymorphism analysis is carried out for the κ-casein gene by restriction fragment length polymorphism (RFLP), by amplifying a fragment comprising nucleotide 5345 of the κ-casein gene and by digesting the product of said amplification with the restriction enzyme HinfI.

5. An identification method according to claim 4, in which the κ-casein gene fragment comprising nucleotide 5345 is amplified with the following primers:

   5'-ATCATTTATGGCCATTCCACCAAAG-3' (SEQ ID No: 1) and
   5'-GCCCATTTCGCCTTCTCTGTAACAGA-3' (SEQ ID No: 2).

6. An identification method according to any one of claims 1 to 3, in which said polymorphism analysis is implemented for the κ-casein gene by allele-specific amplification and/or detection.

7. An identification method according to claim 6, in which allele-specific amplification of the κ-casein gene is carried out using the following primers:

   CCGAAGCAGTAGAGAGCACTGTAG (SEQ ID No: 3);
   TCTCAGGTGGGCTCTCAATAACTT (SEQ ID No: 4);

And the following probes:

Vic: TACTCTAGAAGATTCTC (SEQ ID No: 5);
Fam: TACTCTAGAAGCTTCTC (SEQ ID No: 6).

8. An identification method according to any one of claims 1 to 7, in which said polymorphism analysis is carried out for the Pit-1 gene by restriction fragment polymorphism analysis (RFLP), by amplifying a fragment comprising nucleotide 1178 of the Pit-1 gene and digesting the product of said amplification with the restriction enzyme HinfI.

9. An identification method according to claim 8, in which the Pit-1 gene fragment comprising nucleotide 1178 is amplified with the following primers:

5'-AAACCATCATCTCCCTTCTT-3' (SEQ ID No: 7);
5'-AATGTACAATGTGCCTTCTGAG-3' (SEQ ID No: 8).

10. An identification method according to any one of claims 1 to 7, in which said polymorphism analysis is carried out for the Pit-1 gene by allele-specific amplification and/or detection.

11. An identification method according to claim 10, in which allele-specific amplification of the Pit-1 gene is carried out at exon 6, using the following primers:

5'-CAGAGAGAAAAACGGGTGAAGACAAGCATG-3' (SEQ ID No: 9);
specific for allele B;
5'-CAGAGAGAAAAACGGGTGAAGACAAGCATA-3' (SEQ ID No: 10); and
specific for allele A; and
5'-AGATAGAGGGAAAGATATAGTGAAAGGGACAG-3' (SEQ ID No: 11);
as the reverse primer.

12. An identification method according to claim 10, in which allele-specific amplification of the Pit-1 gene is carried out at exon 2 using the following primers:

5'-C TGC CAT CAC GCC ATA GTT C-3' (SEQ ID No: 12), specific for allele C;
5'- C TGC CAT CAC GCC ATA GTT T-3' (SEQ ID No: 13), specific for allele T; and
5'-CA ACA GGA CTT CAT TAT TCT GTT CCT CAT TAT TCT GTT CCT T -3' (SEQ ID No: 14) as the reverse primer.

13. An identification method according to claim 10, in which allele-specific amplification of the Pit-1 gene is carried out at exon 6 using the following primers:

PIT-1 F primer: CATTCGAGATGCTCCTTAGAAATAGTAA (SEQ ID No: 15);
PIT-1R primer: GTTTTGTAACCGAAGGCAGAGAGA (SEQ ID No: 16);
PIT-1MGB FAM probe: AACTCTGATTTAGGCTTG (for allele A) (SEQ ID No: 17); and
PIT-1MGB VIC probe: AACTCTGATTCAGGCTT (for allele B) (SEQ ID No: 18).

14. A kit for identifying a genotype indicative of advantageous milk production traits in cattle, comprising oligonucleotides for amplifying a fragment comprising nucleotide 1178 of the Pit-1 gene, oligonucleotides for amplifying a fragment comprising nucleotide 5345 of the κ-casein gene, and the restriction enzyme HinfI.

15. A kit according to claim 14, in which the oligonucleotides for amplifying a fragment comprising nucleotide 1178 of the Pit-1 gene are constituted by the following primers:

5'-AAACCATCATCTCCCTTCTT-3' (SEQ ID No: 7); and
5'-AATGTACAATGTGCCTTCTGAG-3' (SEQ ID No: 8);

and the oligonucleotides for amplifying a fragment comprising nucleotide 5345 of the κ-casein gene are constituted by the following primers:

5'-ATCATTTATGGCCATTCCACCAAAG-3' (SEQ ID No: 1) and

5'-GCCCATTTCGCCTTCTCTGTAACAGA-3' (SEQ ID No: 2).

**16.** A kit for identifying a genotype indicative of advantageous milk production traits in cattle, comprising oligonucleotides for carrying out allele-specific amplification and/or detection of a fragment of the Pit-1 gene, and oligonucleotides for carrying out allele-specific amplification and/or detection of a fragment of the κ-casein gene.

**17.** A kit according to claim 16, in which the oligonucleotides for allele-specific amplification of a fragment of the Pit-1 gene are the following primers:

5'-CAGAGAGAAAAACGGGTGAAGACAAGCATG-3' (SEQ ID No: 9); specific for allele B;
5'-CAGAGAGAAAAACGGGTGAAGACAAGCATA-3' (SEQ ID No: 10); specific for allele A; and
5'-AGATAGAGGGAAAGATATAGTGAAAGGGACAG-3' (SEQ ID No: 11);
as the reverse primer.

**18.** A kit according to claim 16, in which the oligonucleotides for allele-specific amplification of a fragment of the Pit-1 gene are the following primers:

5'-C TGC CAT CAC GCC ATA GTT C-3' (SEQ ID No: 12), specific for allele C;
5'- C TGC CAT CAC GCC ATA GTT T-3' (SEQ ID No: 13), specific for allele T; and
5'-CA ACA GGA CTT CAT TAT TCT GTT CCT CAT TAT TCT GTT CCT T -3' (SEQ ID No: 14) as the reverse primer.

**19.** A kit according to claim 16, in which the oligonucleotides for allele-specific detection of a fragment of the Pit-1 gene are the following primers:

PIT-1 F primer: CATTCGAGATGCTCCTTAGAAATAGTAA (SEQ ID No: 15);
PIT-1R primer: GTTTTGTAACCGAAGGCAGAGAGA (SEQ ID No: 16);

and the following probes:

PIT-1MGB FAM probe: AACTCTGATTTAGGCTTG (for allele A) (SEQ ID No: 17); and
PIT-1MGB VIC probe: AACTCTGATTCAGGCTT (for allele B) (SEQ ID No: 18).

**20.** A kit according to claim 16, in which the oligonucleotides for allele-specific detection of a fragment of the κ-casein gene are the following primers:

CCGAAGCAGTAGAGAGCACTGTAG (SEQ ID No: 3);
TCTCAGGTGGGCTCTCAATAACTT (SEQ ID No: 4);

and the following probes:

Vic: TACTCTAGAAGATTCTC (SEQ ID No: 5);
Fam: TACTCTAGAAGCTTCTC (SEQ ID No: 6).

**Patentansprüche**

**1.** Verfahren zur Identifizierung eines Rindes mit einem Genotyp, der auf vorteilhafte Milchproduktions-Merkmale hinweist, umfassend das Analysieren, in einer biologischen Probe aus dem Rind, des Polymorphismus der Pit-1- und κ-Casein-Gene des Rinds, wobei die gleichzeitige Gegenwart von Allel A und/oder T des Pit-1-Gens und von Allel B des κ-Casein-Gens auf ein hohes Potential für die Milchproduktion und Proteinproduktion in dem Rind hinweist, wobei das Allel A des Pit-1-Gens durch die Gegenwart von Adenin an Position 1178 des Pit-1-Gens **gekennzeichnet** ist,
wobei das Allel T des Pit-1-Gens durch die Gegenwart von Guanin an Position 195 des Pit-1-Gens **gekennzeichnet** ist,
wobei das Allel B des κ-Casein-Gens durch die Gegenwart von Cytosin an Position 5345 des κ-Casein-Gens **gekennzeichnet** ist.

**2.** Identifizierungsverfahren gemäß Anspruch 1, wobei die Gegenwart des Allels B des κ-Caseins außerdem auf eine laktodynamographische Milchqualität hinweist, die für die Käseproduktion günstig ist.

**3.** Identifizierungsverfahren gemäß Anspruch 1 oder 2, wobei die biologische Probe Haarfollikelzellen-DNA von dem Rind umfasst.

**4.** Identifizierungsverfahren gemäß mindestens einem der Ansprüche 1 bis 3, wobei die Polymorphismusanalyse für das κ-Casein-Gen durch Restriktionsfragmentlängenpolymorphismus (RFLP), durch Amplifizieren eines Fragments, welches das Nukleotid 5345 des κ-Casein-Gens umfasst, und durch den Verdau des Produkts der Amplifikation mit dem Restriktionsenzym Hinfl durchgeführt wird.

**5.** Identifizierungsverfahren gemäß Anspruch 4, wobei das κ-Casein-Genfragment, welches das Nukleotid 5345 umfasst, mit den folgenden Primem amplifiziert wird:

> 5'-ATCATTTATGGCCATTCCACCAAAG-3' (SEQ ID Nr.: 1) und
> 5'-GCCCATTTCGCCTTCTCTGTAACAGA-3' (SEQ ID Nr.: 2).

**6.** Identifizierungsverfahren gemäß mindestens einem der Ansprüche 1 bis 3, worin die Polymorphismusanalyse für das κ-Casein-Gen durch allelspezifische Amplifikation und/oder Detektion durchgeführt wird.

**7.** Identifizierungsverfahren gemäß Anspruch 6, wobei die allelspezifische Amplifikation des κ-Casein-Gens durchgeführt wird unter Verwendung der folgenden Primer:

> CCGAAGCAGTAGAGAGCACTGTAG (SEQ ID Nr.: 3);
> TCTCAGGTGGGCTCTCAATAACTT (SEQ ID Nr.: 4);

> und der folgenden Sonden:

> Vic: TACTCTAGAAGATTCTC (SEQ ID Nr.: 5);
> Fam: TACTCTAGAAGCTTCTC (SEQ ID Nr.: 6).

**8.** Identifizierungsverfahren gemäß mindestens einem der Ansprüche 1 bis 7, worin die Polymorphismusanalyse für das Pit-1-Gen durch Restriktionsfragment-Polymorphismusanalyse (RFLP), durch Amplifizieren eines Fragments, welches das Nukleotid 1178 des Pit-1-Gens umfasst, und durch den Verdau des Produkts der Amplifikation mit dem Restriktionsenzym Hinfl durchgeführt wird.

**9.** Identifizierungsverfahren gemäß Anspruch 8, worin das Pit-1-Genfragment, welches das Nukleotid 1178 umfasst, mit den folgenden Primem amplifiziert wird:

> 5'-AAACCATCATCTCCCTTCTT-3' (SEQ ID Nr.: 7);
> 5'-AATGTACAATGTGCCTTCTGAG-3' (SEQ ID Nr.: 8).

**10.** Identifizierungsverfahren gemäß mindestens einem der Ansprüche 1 bis 7, worin die Polymorphismusanalyse für das Pit-1-Gen durch allelspezifische Amplifikation und/oder Detektion durchgeführt wird.

**11.** Identifizierungsverfahren gemäß Anspruch 10, worin die allelspezifische Amplifikation des Pit-1-Gens am Exon 6 unter Verwendung der folgenden Primer durchgeführt wird:

> 5'-CAGAGAGAAAAACGGGTGAAGACAAGCATG-3' (SEQ ID Nr.: 9);
> spezifisch für Allel B;
> 5'-CAGAGAGAAAAACGGGTGAAGACAAGCATA-3' (SEQ ID Nr.: 10); und
> spezifisch für Allel A; und
> 5'-AGATAGAGGGAAAGATATAGTGAAAGGGACAG-3' (SEQ ID Nr.: 11);
> als Rückwärtsprimer.

**12.** Identifizierungsverfahren gemäß Anspruch 10, worin die allelspezifische Amplifikation des Pit-1-Gens am Exon 2 unter Verwendung der folgenden Primer durchgeführt wird:

5'-C TGC CAT CAC GCC ATA GTT C-3' (SEQ ID Nr.: 12), spezifisch für Allel C;
5'- C TGC CAT CAC GCC ATA GTT T-3' (SEQ ID Nr.: 13), spezifisch für Allel T; und
5'-CA ACA GGA CTT CAT TAT TCT GTT CCT CAT TAT TCT GTT CCT T -3' (SEQ ID Nr.: 14) als Rückwärtsprimer.

13. Identifizierungsverfahren gemäß Anspruch 10, worin die allelspezifische Amplifikation des Pit-1-Gens am Exon 6 unter Verwendung der folgenden Primer durchgeführt wird:

PIT-1 F Primer: CATTCGAGATGCTCCTTAGAAATAGTAA (SEQ ID Nr.: 15);
PIT-1R Primer: GTTTTGTAACCGAAGGCAGAGAGA (SEQ ID Nr.: 16);
PIT-1MGB FAM-Sonde: AACTCTGATTTAGGCTTG (für Allel A) (SEQ ID Nr.: 17); und
PIT-1MGB VIC-Sonde: AACTCTGATTCAGGCTT (für Allel B) (SEQ ID Nr.: 18).

14. Kit zum Identifizieren eines Genotyps, der auf vorteilhafte Milchproduktions-Merkmale bei Rindvieh hinweist, umfassend Oligonukleotide zum Amplifizieren eines Fragments, welches das Nukleotid 1178 des Pit-1-Gens umfasst, Oligonukleotide zum Amplifizieren eines Fragments, welches das Nukleotid 5345 des κ-Casein-Gens umfasst, und das Restriktionsenzym HinfI.

15. Kit gemäß Anspruch 14, in welchem die Oligonukleotide zum Amplifizieren eines Fragments, welches das Nukleotid 1178 des Pit-1-Gens umfasst, durch die folgenden Primer aufgebaut werden:

5'-AAACCATCATCTCCCTTCTT-3' (SEQ ID Nr.: 7); und
5'-AATGTACAATGTGCCTTCTGAG-3' (SEQ ID Nr.: 8);

und die Oligonukleotide zum Amplifizieren eines Fragments, welches das Nukleotid 5345 des κ-Casein-Gens umfasst, durch die folgenden Primer aufgebaut werden:

5'-ATCATTTATGGCCATTCCACCAAAG-3' (SEQ ID Nr.: 1) und
5'-GCCCATTTCGCCTTCTCTGTAACAGA-3' (SEQ ID Nr.: 2).

16. Kit zum Identifizieren eines Genotyps, der auf vorteilhafte Milchproduktions-Merkmale bei Rindvieh hinweist, umfassend Oligonukleotide zur Durchführung von allelspezifischer Amplifikation und/oder Detektion eines Fragments des Pit-1-Gens und Oligonukleotide zur Durchführung von allelspezifischer Amplifikation und/oder Detektion eines Fragments des κ-Casein-Gens.

17. Kit gemäß Anspruch 16, in welchem die Oligonukleotide zur allelspezifischen Amplifikation eines Fragments des Pit-1-Gens die folgenden Primer sind:

5'-CAGAGAGAAAAACGGGTGAAGACAAGCATG-3' (SEQ ID Nr.: 9);
spezifisch für Allel B;
5'-CAGAGAGAAAAACGGGTGAAGACAAGCATA-3' (SEQ ID Nr.: 10);
spezifisch für Allel A; und
5'-AGATAGAGGGAAAGATATAGTGAAAGGGACAG-3' (SEQ ID Nr.: 11);
als Rückwärtsprimer.

18. Kit gemäß Anspruch 16, in welchem die Oligonukleotide für die allelspezifische Amplifikation eines Fragments des Pit-1-Gens die folgenden Primer sind:

5'-C TGC CAT CAC GCC ATA GTT C-3' (SEQ ID Nr.: 12), spezifisch für Allel C;
5'- C TGC CAT CAC GCC ATA GTT T-3' (SEQ ID Nr.: 13), spezifisch für Allel T; und
5'-CA ACA GGA CTT CAT TAT TCT GTT CCT CAT TAT TCT GTT CCT T -3' (SEQ ID Nr.: 14) als Rückwärtsprimer.

19. Kit gemäß Anspruch 16, in welchem die Oligonukleotide für die allelspezifische Detektion eines Fragments des Pit-1-Gens die folgenden Primer:

PIT-1 F-Primer: CATTCGAGATGCTCCTTAGAAATAGTAA (SEQ ID Nr.: 15);
PIT-1R-Primer: GTTTTGTAACCGAAGGCAGAGAGA (SEQ ID Nr.: 16);

und die folgenden Sonden sind:

PIT-1MGB FAM-Sonde: AACTCTGATTTAGGCTTG (für Allel A) (SEQ ID Nr.: 17); und
PIT-1MGB VIC-Sonde: AACTCTGATTCAGGCTT (für Allel B) (SEQ ID Nr.: 18).

**20.** Kit gemäß Anspruch 16, in welchem die Oligonukleotide für die allelspezifische Detektion eines Fragments des κ-Casein-Gens die folgenden Primer:

CCGAAGCAGTAGAGAGCACTGTAG (SEQ ID Nr.: 3);
TCTCAGGTGGGCTCTCAATAACTT (SEQ ID Nr.: 4);

und die folgenden Sonden sind:

Vic: TACTCTAGAAGATTCTC (SEQ ID Nr.: 5);
Fam: TACTCTAGAAGCTTCTC (SEQ ID Nr.: 6).

**Revendications**

**1.** Procédé pour identifier un bovin ayant un génotype qui est indicateur de traits de production laitière avantageux, comprenant l'analyse dans un échantillon biologique provenant dudit bovin du polymorphisme des gènes Pit-1 et de caséine-κ dudit bovin, dans lequel la présence simultanée de l'allèle A et/ou T du gène Pit-1 et l'allèle B du gène de caséine-κ est indicateur d'un fort potentiel de production laitière et de production de protéines chez ledit bovin, ledit allèle A du gène Pit-1 étant **caractérisé par** la présence d'une adénine à la position 1178 du gène Pit-1, ledit allèle T du gène Pit-1 étant **caractérisé par** la présence d'une guanine à la position 195 du gène Pit-1, ledit allèle B du gène de κ-caséine étant **caractérisé par** la présence d'une cytosine à la position 5345 du gène de caséine-K.

**2.** Procédé d'identification selon la revendication 1, dans lequel la présence de l'allèle B de la caséine-κ est également indicatrice d'une qualité de lait lactodynamographique qui est favorable à la production de fromage.

**3.** Procédé d'identification selon la revendication 1 ou 2, dans lequel ledit échantillon biologique comprend de l'ADN cellulaire de follicule pileux dudit bovin.

**4.** Procédé d'identification selon l'une quelconque des revendications 1 à 3, dans lequel ladite analyse de polymorphisme est réalisée pour le gène de caséine-κ par polymorphisme de longueur des fragments de restriction (RFLP), par amplification d'un fragment comprenant le nucléotide 5345 du gène de caséine-κ et par digestion du produit de ladite amplification avec l'enzyme de restriction HinfI.

**5.** Procédé d'identification selon la revendication 4, dans lequel le fragment du gène de caséine-κ comprenant le nucléotide 5345 est amplifié avec les amorces suivantes :

5'-ATCATTTATGGCCATTCCACCAAAG-3' (SEQ ID No: 1) et
5'-GCCCATTTCGCCTTCTCTGTAACAGA-3' (SEQ ID No: 2).

**6.** Procédé d'identification selon l'une quelconque des revendications 1 à 3, dans lequel ladite analyse de polymorphisme est mise en oeuvre pour le gène de caséine-κ par une amplification et/ou une détection spécifique d'un allèle.

**7.** Procédé d'identification selon la revendication 6, dans lequel une amplification spécifique d'un allèle du gène de caséine-κ est réalisée en utilisant les amorces suivantes :

CCGAAGCAGTAGAGAGCACTGTAG (SEQ ID No: 3) ;
TCTCAGGTGGGCTCTCAATAACTT (SEQ ID No: 4) ;

et les sondes suivantes :

Vic: TACTCTAGAAGATTCTC (SEQ ID No: 5) ;

Fam: TACTCTAGAAGCTTCTC (SEQ ID No: 6).

8. Procédé d'identification selon l'une quelconque des revendications 1 à 7, dans lequel ladite analyse de polymorphisme est réalisée pour le gène Pit-1 par analyse du polymorphisme des fragments de restriction (RFLP), par amplification d'un fragment comprenant le nucléotide 1178 du gène Pit-1 et digestion du produit de ladite amplification avec l'enzyme de restriction HinfI.

9. Procédé d'identification selon la revendication 8, dans lequel le fragment de gène Pit-1 comprenant le nucléotide 1178 est amplifié avec les amorces suivantes :

   5'-AAACCATCATCTCCCTTCTT-3' (SEQ ID No: 7) ;
   5'-AATGTACAATGTGCCTTCTGAG-3' (SEQ ID No: 8).

10. Procédé d'identification selon l'une quelconque des revendications 1 à 7, dans lequel ladite analyse de polymorphisme est réalisée pour le gène Pit-1 par une amplification et/ou une détection spécifique d'un allèle.

11. Procédé d'identification selon la revendication 10, dans lequel une amplification spécifique d'un allèle du gène Pit-1 est réalisée au niveau de l'exon 6, en utilisant les amorces suivantes :

   5'-CAGAGAGAAAAACGGGTGAAGACAAGCATG-3' (SEQ ID No: 9) ; spécifique de l'allèle B ;
   5'-CAGAGAGAAAAACGGGTGAAGACAAGCATA-3' (SEQ ID No: 10) ; et spécifique de l'allèle A ; et
   5'-AGATAGAGGGAAAGATATAGTGAAAGGGACAG-3' (SEQ ID No: 11) ; comme amorce antisens.

12. Procédé d'identification selon la revendication 10, dans lequel une amplification spécifique d'un allèle du gène Pit-1 est réalisée au niveau de l'exon 2 en utilisant les amorces suivantes :

   5'-C TGC CAT CAC GCC ATA GTT C-3' (SEQ ID No: 12) ; spécifique de l'allèle C ;
   5'- C TGC CAT CAC GCC ATA GTT T-3' (SEQ ID No: 13) ; spécifique de l'allèle T ; et
   5'-CA ACA GGA CTT CAT TAT TCT GTT CCT CAT TAT TCT GTT CCT T -3' (SEQ ID No: 14) comme amorce antisens.

13. Procédé d'identification selon la revendication 10, dans lequel une amplification spécifique d'un allèle du gène Pit-1 est réalisée au niveau de l'exon 6 en utilisant les amorces suivantes :

   amorce PIT-1 F : CATTCGAGATGCTCCTTAGAAATAGTAA (SEQ ID No: 15) ;
   amorce PIT-1R : GTTTTGTAACCGAAGGCAGAGAGA (SEQ ID No: 16) ;
   sonde PIT-1MGB FAM : AACTCTGATTTAGGCTTG (pour l'allèle A) (SEQ ID No: 17) ; et
   sonde PIT-1MGB VIC : AACTCTGATTCAGGCTT (pour l'allèle B) (SEQ ID No: 18).

14. Kit pour identifier un génotype indicateur de traits de production laitière avantageux chez du bétail, comprenant des oligonucléotides pour amplifier un fragment comprenant le nucléotide 1178 du gène Pit-1, des oligonucléotides pour amplifier un fragment comprenant le nucléotide 5345 du gène de caséine-κ, et l'enzyme de restriction HinfI.

15. Kit selon la revendication 14, dans lequel les oligonucléotides pour amplifier un fragment comprenant le nucléotide 1178 du gène Pit-1 sont constitués par les amorces suivantes :

   5'-AAACCATCATCTCCCTTCTT-3' (SEQ ID No: 7) ; et
   5'-AATGTACAATGTGCCTTCTGAG-3' (SEQ ID No: 8) ;

   et les oligonucléotides pour amplifier un fragment comprenant le nucléotide 5345 du gène de caséine-κ sont constitués par les amorces suivantes :

   5'-ATCATTTATGGCCATTCCACCAAAG-3' (SEQ ID No: 1) et
   5'-GCCCATTTCGCCTTCTCTGTAACAGA-3' (SEQ ID No: 2).

16. Kit pour identifier un génotype indicateur de traits de production laitière avantageux chez du bétail, comprenant des oligonucléotides pour réaliser une amplification et/ou une détection spécifique d'un allèle d'un fragment du gène Pit-1, et des oligonucléotides pour réaliser une amplification et/ou une détection spécifique d'un allèle d'un fragment du gène de caséine-κ.

17. Kit selon la revendication 16, dans lequel les oligonucléotides pour une amplification spécifique d'un allèle d'un fragment du gène Pit-1 sont les amorces suivantes :

5'-CAGAGAGAAAAACGGGTGAAGACAAGCATG-3' (SEQ ID No: 9) ; spécifique de l'allèle B ;
5'-CAGAGAGAAAAACGGGTGAAGACAAGCATA-3' (SEQ ID No: 10) ; spécifique de l'allèle A ; et
5'-AGATAGAGGGAAAGATATAGTGAAAGGGACAG-3' (SEQ ID No: 11) ; comme amorce antisens.

18. Kit selon la revendication 16, dans lequel les oligonucléotides pour une amplification spécifique d'un allèle d'un fragment du gène Pit-1 sont les amorces suivantes :

5'-C TGC CAT CAC GCC ATA GTT C-3' (SEQ ID No: 12), spécifique de l'allèle C ;
5'- C TGC CAT CAC GCC ATA GTT T-3' (SEQ ID No: 13), spécifique de l'allèle T ; et
5'-CA ACA GGA CTT CAT TAT TCT GTT CCT CAT TAT TCT GTT CCT T -3' (SEQ ID No: 14) comme amorce antisens.

19. Kit selon la revendication 16, dans lequel les oligonucléotides pour une détection spécifique d'un allèle d'un fragment du gène Pit-1 sont les amorces suivantes :

amorce PIT-1 F : CATTCGAGATGCTCCTTAGAAATAGTAA (SEQ ID No: 15) ;
amorce PIT-1R : GTTTTGTAACCGAAGGCAGAGAGA (SEQ ID No: 16) ;

et les sondes suivantes :

sonde PIT-1MGB FAM : AACTCTGATTTAGGCTTG (pour l'allèle A) (SEQ ID No: 17) ; et
sonde PIT-1MGB VIC : AACTCTGATTCAGGCTT (pour l'allèle B) (SEQ ID No: 18).

20. Kit selon la revendication 16, dans lequel les oligonucléotides pour une détection spécifique d'un allèle d'un fragment du gène de caséine-κ sont les amorces suivantes :

CCGAAGCAGTAGAGAGCACTGTAG (SEQ ID No: 3) ;
TCTCAGGTGGGCTCTCAATAACTT (SEQ ID No: 4) ;

et les sondes suivantes :

Vic : TACTCTAGAAGATTCTC (SEQ ID No: 5) ;
Fam : TACTCTAGAAGCTTCTC (SEQ ID No: 6).

AMORCE

320 pb

FIGURE 1

Amorce Pit R    CA ACA GGA CTT CAT TAT TCT GTT CCT CAT TAT TCT GTT CCT T
*5'...GTG ATG TCC ACA GCA ACA GGA CTT CAT TAT TCT GTT CCT CAT TAT TCT GTT CCT TTC*

*TGT CAT TAT GGA AAC CAG TCA TCG ACC TAT GGC GTG ATG GCA GGG AGC TTA ACC..3'*
C TTG ATA CCG CAC TAC CGT C Amorce Pit C
T TTG ATA CCG CAC TAC CGT C Amorce Pit T

FIGURE 2

AMORCES    C    T    C    T    C    T

→ **Contrôle interne de la PCR**

→ **Fragment de l'exon 2 de Pit-1**

FIGURE 3

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9803677 A
- EP 96401634 A
- FR 0203026

**Non-patent literature cited in the description**

- **Sambrook et al.** Molecular Cloning - A Laboratory Manual. Cold Spring Harbor Laboratory Press
- **Alexander et al.** *Eur. J. Biochem.,* 1988, vol. 178 (2), 395-401
- **Bovenhuis S. ; Van Arendonk J. ; Korver S.** Association between milk protein polymorph isms and milk traits. *J. Dairy Sci.,* 1992, vol. 75, 2549-2559
- **Chilliard et al.** Biotechnology in Animal Husbandry. Kluwer academic publishing, May 2001, 65-97
- **Formaggioni et al.** Milk protein polymorphism: detection and diffusion of the genetic variants in bos genus. *Annali della Facolta di Medicina Veterinaria,* 1999, vol. XIX
- **Mao I. ; Buttanozzi L.G. ; Aleandri R.** Effects of polymorphic milk protein genes on milk yield and composition traits in Holstein cattle. *Acta Agric. Scand., Sect. A, Animal Sci.,* 1992, vol. 42, 1-7
- **Martinet ; Houdebine.** Biologie de la lactation Editions Inserm. 1993, 587
- **Ron M. ; Yoffe O. ; Ezra E. ; Medrano J.F. ; Weller.** Determination of effects of milk protein genotype on production traits of Israeli Holsteins. *J. Dairy Sci.,* 1994, vol. 77, 1106-1113
- **Sambrook et al.** Molecular Cloning - A laboratory manual. Cold Spring Harbor Laboratory Press
- **Van Eenennaam A. ; Medrano J.F.** Milk protein polymorphisms in California dairy cattle. *J. Dairy Sci.,* 1991, vol. 74, 1730-1742

SEQUENCE LISTING

<110> ARYSTA LIFESCIENCE CORPORATION
PATRIMOINE DE LA FACULTE UNIVERSITAIRE DES SCIENCES AGRONOMIQUES DE GEMBLOUX

<120> METHOD FOR IDENTIFYING ANIMALS LIKELY TO HAVE GOOD MILK PRODUCTION QUALITIES BY ANALYZING THE POLYMORPHISM OF THE PIT-1 AND KAPPA-CASEIN GENES

<130> B5163A - AD/VMA/VG

<140> New International Patent Application
<141> 2003-03-11

<150> FR 0203026<151> 2002-03-11

<160> 20

<170> PatentIn version 3.1

<210> 1
<211> 25
<212> DNA
<213> PRIMER

<400> 1

```
atcatttatg gccattccac caaag
25
```

<210> 2
<211> 26
<212> DNA
<213> PRIMER

<400> 2

```
gcccatttcg ccttctctgt aacaga
26
```

<210> 3
<211> 24
<212> DNA
<213> PRIMER

<400> 3

```
ccgaagcagt agagagcact gtag
24
```

<210> 4
<211> 24
<212> DNA
<213> PRIMER

<400> 4

```
tctcaggtgg gctctcaata actt
24
```

<210> 5
<211> 17
<212> DNA
<213> PROBE

<400> 5

```
tactctagaa gattctc
         17
```

<210> 6
<211> 17
<212> DNA
<213> PROBE

<400> 6

```
tactctagaa gcttctc
         17
```

<210> 7
<211> 20
<212> DNA
<213> PRIMER

<400> 7

```
aaaccatcat ctcccttctt
         20
```

<210> 8
<211> 22
<212> DNA
<213> PRIMER

<400> 8

```
aatgtacaat gtgccttctg ag
         22
```

<210> 9
<211> 30
<212> DNA
<213> PRIMER

<400> 9

```
cagagagaaa aacgggtgaa gacaagcatg
         30
```

<210> 10
<211> 30
<212> DNA
<213> PRIMER

<400> 10

```
cagagagaaa aacgggtgaa gacaagcata
         30
```

<210> 11
<211> 32
<212> DNA
<213> PRIMER

<400> 11

agatagaggg aaagatatag tgaaagggac ag
32

<210> 12
<211> 20
<212> DNA
<213> PRIMER

<220>
<221> misc_feature
<222> (1)..(20)
<223> FIGURE 2

<400> 12

ctgccatcac gccatagttc
20

<210> 13
<211> 20
<212> DNA
<213> PRIMER

<220>
<221> misc_feature
<222> (1)..(20)
<223> FIGURE 2

<400> 13

ctgccatcac gccatagttt
20

<210> 14
<211> 42
<212> DNA
<213> PRIMER

<220>
<221> misc_feature
<222> (1)..(42)
<223> FIGURE 2

<400> 14

caacaggact tcattattct gttcctcatt attctgttcc tt
42

<210> 15
<211> 28
<212> DNA
<213> PRIMER

<400> 15

cattcgagat gctccttaga aatagtaa         28

<210> 16
<211> 24
<212> DNA
<213> PRIMER

<400> 16

gttttgtaac cgaaggcaga gaga         24

<210> 17
<211> 18
<212> DNA
<213> PROBE

<400> 17

aactctgatt taggcttg         18

<210> 18
<211> 17
<212> DNA
<213> PROBE

<400> 18

aactctgatt caggctt         17

<210> 19
<211> 57
<212> DNA
<213> GENOMIC DNA

<220>
<221> misc_feature
<222> (1)..(57)
<223> FIGURE 2

<400> 19

```
gtgatgtcca cagcaacagg acttcattat tctgttcctc attattctgt tcctttc
                57
```

<210> 20
<211> 54
<212> DNA
<213> GENOMIC DNA

<220>
<221> misc_feature
<222> (1)..(54)
<223> FIGURE 2

<400> 20

```
tgtcattatg gaaaccagtc atcgacctat ggcgtgatgg cagggagctt aacc
                54
```